(19) **Europäisches Patentamt / European Patent Office / Office européen des brevets**

(11) **EP 3 673 990 A1**

(12) **EUROPEAN PATENT APPLICATION**
published in accordance with Art. 153(4) EPC

(43) Date of publication:
01.07.2020 Bulletin 2020/27

(51) Int Cl.:
B01J 20/285 (2006.01)   B01D 15/36 (2006.01)
B01J 41/04 (2017.01)   B01J 41/14 (2006.01)
B01J 41/20 (2006.01)   B01J 47/00 (2017.01)
C12N 15/00 (2006.01)   C12Q 1/68 (2018.01)
G01N 30/02 (2006.01)   G01N 30/88 (2006.01)

(21) Application number: 18847842.4

(22) Date of filing: 27.08.2018

(86) International application number:
PCT/JP2018/031539

(87) International publication number:
WO 2019/039613 (28.02.2019 Gazette 2019/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.08.2017   JP 2017161989

(71) Applicant: Sekisui Medical Co., Ltd.
Tokyo 103-0027 (JP)

(72) Inventors:
• YOTANI, Takuya
Tokyo 103-0027 (JP)
• MATSUDA, Kohei
Tokyo 103-0027 (JP)

(74) Representative: Blodig, Wolfgang
Wächtershäuser & Hartz
Patentanwaltspartnerschaft mbB
Weinstrasse 8
80333 München (DE)

(54) **CHROMATOGRAPHY PACKING FOR SEPARATION AND/OR DETECTION OF METHYLATED DNA**

(57)    Provided is a chromatography packing that can detect methylated DNA with high accuracy. An ion-exchange chromatography packing for separation and/or detection of methylated DNA, containing a base particle consisting of a hydrophobic crosslinked copolymer particle having a cationic functional group on a surface, wherein a hydrophobic crosslinked copolymer contains a divinyl aromatic monomer.

EP 3 673 990 A1

## Description

Field of the Invention

The present invention relates to a chromatography packing for separation and/or detection of methylated DNA.

Background of the Invention

DNA methylation is one of the most commonly observed epigenetic changes associated with carcinogenesis. As a characteristic epigenetic abnormality in cancer cells, abnormal DNA methylation of CpG island has been known. A CpG island is a region in which a two-base sequence of cytosine (C)-guanine (G) through a phosphodiester bond (p) appears at a high frequency, and often exists in a promoter region upstream of a gene. Abnormal DNA methylation of CpG islands is involved in carcinogenesis through inactivation of tumor suppressor genes.

As a method for analyzing methylated DNA, a method using a bisulfite method has already been established. If a single-stranded DNA is treated with bisulfite, cytosine is converted into uracil, while methylated cytosine remains cytosine. Therefore, if DNA treated with bisulfite is subjected to PCR, methylated cytosine is amplified with cytosine as it is; whereas, non-methylated cytosine is amplified by replacing uracil with thymine, and thus DNA methylation results in differences in the sequence of PCR amplification products. In the conventional analysis method for methylated DNA, it was common to detect the difference in the sequence of this PCR amplification product by electrophoresis or sequencing. However, in these methods, there is a disadvantage in that labor and time for electrophoresis or sequencing are required.

In the fields of biochemistry and medicine, ion-exchange chromatography has been widely used as a method for easily and accurately separating and detecting biopolymers such as nucleic acids, proteins, and polysaccharides. In a case of separating nucleic acids by ion-exchange chromatography, in general, anion-exchange-chromatography that separates nucleic acids by utilizing a negative charge of phosphate in the nucleic acid molecule is used. Examples of the cationic functional group used in the column packing for anion-exchange chromatography include a weak cationic group such as a diethylaminoethyl group and a strong cationic group such as a quaternary ammonium group. Furthermore, Patent Literature 1 discloses that a single base difference between 20-mer oligonucleotides can be detected by ion-exchange chromatography using a column packing having both a strong cationic group and a weak cationic group as a functional group.

Patent Literature 2 discloses a method for detecting methylation of sample DNA, performed in such a manner that sample DNA is treated with bisulfite and then amplified by PCR, and the amplification product is subjected to ion-exchange chromatography using a column packing having both a strong cationic group and a weak cationic group as a functional group. This is an excellent method that enables analysis of methylated DNA in a simple and short time compared to the conventional method using electrophoresis or sequencing.

Citation List

Patent Literature

Patent Literature 1: WO 2012/108516 A
Patent Literature 2: WO 2014/136930 A

Summary of the Invention

Problem to be solved by the Invention

In the detection method for methylated DNA using ion-exchange chromatography as disclosed in Patent Literature 2, the degree of separation between the peak of methylated DNA and the peak of non-methylated DNA in chromatography is required to be more increased in order to improve detection accuracy. The present invention provides a chromatography packing capable of separating and/or detecting methylated DNA with high accuracy.

Means for solving the problem

The present invention provides the following.

[1] An ion-exchange chromatography packing for separation and/or detection of methylated DNA, containing a base particle consisting of a hydrophobic crosslinked copolymer particle and having a cationic functional group on a surface, wherein a hydrophobic crosslinked copolymer contains a divinyl aromatic monomer.

[2] The ion-exchange chromatography packing according to [1], wherein the divinyl aromatic monomer is selected from the group consisting of divinylbenzene, divinylnaphthalene, divinylanthracene, divinyltoluene, divinylxylene, and divinylbiphenyl.

[3] The ion-exchange chromatography packing according to [1] or [2], wherein the hydrophobic crosslinked copolymer contains 3% to 15% by mass of the divinyl aromatic monomer in a total mass.

[4] The ion-exchange chromatography packing according to any one of [1] to [3], wherein the hydrophobic crosslinked copolymer contains the divinyl aromatic monomer, a non-aromatic hydrophobic crosslinked monomer having two or more vinyl groups, and a monomer having a reactive functional group.

[5] The ion-exchange chromatography packing according to [4], wherein the reactive functional group is a glycidyl group or an isocyanate group.

[6] The ion-exchange chromatography packing according to [4] or [5], wherein the non-aromatic hydrophobic crosslinked monomer having two or more vinyl groups is at least one selected from the group consisting of diethylene glycol dimethacrylate, triethylene glycol dimethacrylate, tetraethylene glycol dimethacrylate, trimethylol methane trimethacrylate, tetramethylol methane trimethacrylate, diethylene glycol diacrylate, triethylene glycol diacrylate, tetraethylene glycol diacrylate, trimethylol methane triacrylate, and tetramethylol methane triacrylate.

[7] The ion-exchange chromatography packing according to any one of [1] to [6], wherein the hydrophobic crosslinked copolymer contains 3% to 15% by mass of the divinyl aromatic monomer, 40% to 70% by mass of the non-aromatic hydrophobic crosslinked monomer, and 10% to 50% by mass of the monomer having the reactive functional group in the total mass.

[8] The ion-exchange chromatography packing according to any one of [1] to [7], wherein the hydrophobic crosslinked copolymer further contains 0% to 5% by mass of a hydrophobic non-crosslinked monomer in the total mass.

[9] The ion-exchange chromatography packing according to any one of [1] to [8], wherein the cationic functional groups are a strong cationic group and a weak cationic group.

[10] The ion-exchange chromatography packing according to [9], wherein the strong cationic group is a quaternary ammonium group and the weak cationic group is a tertiary amino group.

[11] The ion-exchange chromatography packing according to [9] or [10], wherein the base particle has a polymer layer having the strong cationic group and the weak cationic group on the surface.

[12] An ion-exchange chromatography column for separation and/or detection of methylated DNA containing the ion-exchange chromatography packing according to any one of [1] to [11].

[13] A method for separating and/or detecting methylated DNA using the ion-exchange chromatography packing according to any one of [1] to [11].

Effects of the Invention

The ion-exchange chromatography packing of the present invention enables an increase in the degree of separation of a methylated DNA peak and a non-methylated DNA peak in anion-exchange chromatography, and improves detection accuracy of the methylated DNA.

Brief Description of the Drawings

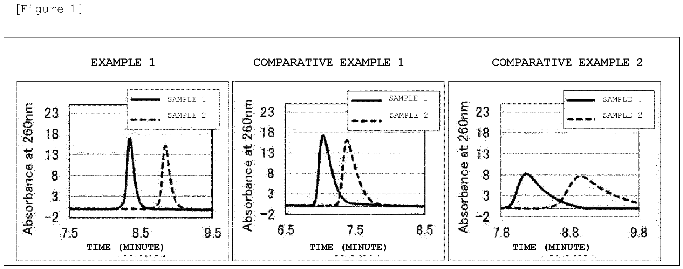

Fig. 1 is a chromatogram of a sample DNA obtained by ion-exchange chromatography analysis using base particles of Example 1 and Comparative Examples 1 and 2.

Description of the Embodiments

The present invention provides an ion-exchange chromatography packing for separation and/or detection of methylated DNA. The packing of the present invention contains a base particle having a cationic functional group on a surface, and is suitably used for anion-exchange chromatography.

The base particle contained in the packing of the present invention contains a hydrophobic crosslinked polymer consisting of a synthetic organic polymer, more specifically, a hydrophobic crosslinked copolymer containing a divinyl aromatic monomer (A). Preferably, the base particle contained in the packing of the present invention consists of the hydrophobic crosslinked copolymer having a cationic functional group on the surface.

In addition to the divinyl aromatic monomer (A), the hydrophobic crosslinked copolymer contains a non-aromatic hydrophobic crosslinked monomer (B) and a monomer (C) having a reactive functional group. Further, it may contain a hydrophobic non-crosslinked monomer (D). Therefore, the hydrophobic crosslinked copolymer may be a hydrophobic crosslinked copolymer obtained by copolymerizing the (A), (B) and (C), or a hydrophobic crosslinked copolymer obtained by copolymerizing the (A), (B), (C), and (D).

Examples of the divinyl aromatic monomer (A) include divinylbenzene, divinylnaphthalene, divinylanthracene, divinyltoluene, divinylxylene, and divinylbiphenyl. The divinyl aromatic (A) exemplified above may be used alone or in combination of any two or more thereof. Preferably, the divinyl aromatic (A) is divinylbenzene.

The non-aromatic hydrophobic crosslinked monomer (B) is not particularly limited as long as it is a non-aromatic compound having two or more vinyl groups in one monomer molecule, and examples thereof include diethylene glycol dimethacrylate, triethylene glycol dimethacrylate, tetraethylene glycol dimethacrylate, trimethylol methane trimethacrylate, tetramethylol methane trimethacrylate, diethylene glycol diacrylate, triethylene glycol diacrylate, tetraethylene glycol diacrylate, trimethylol methane triacrylate, and tetramethylol methane triacrylate. The monomer (B) exemplified above may be used alone or in combination of any two or more thereof. More preferable examples include at least one selected from the group consisting of triethylene glycol dimethacrylate, tetraethylene glycol dimethacrylate, and trimethylolmethane triacrylate.

The reactive functional group in the monomer (C) having the reactive functional group is preferably a glycidyl group or an isocyanate group, and more preferably a glycidyl group. Examples of the monomer (C) include glycidyl methacrylate, glycidyl acrylate, isocyanate ethyl methacrylate, and isocyanate ethyl acrylate, and glycidyl methacrylate is preferable. The monomer (C) exemplified above may be used alone or in combination of any two or more thereof.

The hydrophobic non-crosslinked monomer (D) is not particularly limited as long as it is a non-crosslinked polymerizable organic monomer having hydrophobic properties, and examples thereof include methacrylic acid esters and acrylic acid esters such as methyl methacrylate, methyl acrylate, ethyl methacrylate, ethyl acrylate, butyl methacrylate, butyl acrylate, t-butyl methacrylate, and t-butyl acrylate, and styrene monomers such as styrene and methylstyrene, and combinations thereof.

Examples of preferable combinations of the monomers (A) to (D) constituting the hydrophobic crosslinked copolymer include (A) divinylbenzene, (B) at least one selected from triethylene glycol dimethacrylate, trimethylol methane triacrylate, and pentaerythritol triacrylate, (C) glycidyl methacrylate or isocyanate ethyl methacrylate, and (D) none or styrene. Examples of more preferable combinations include (A) divinylbenzene, (B) triethylene glycol dimethacrylate and trimethylol methane triacrylate, (C) glycidyl methacrylate or isocyanate ethyl methacrylate, and (D) none or styrene. Examples of still more preferable combinations include (A) divinylbenzene, (B) triethylene glycol dimethacrylate and trimethylol methane triacrylate, and (C) glycidyl methacrylate.

The content of the divinyl aromatic monomer (A) in the hydrophobic crosslinked copolymer is preferably 3% to 15% by mass, more preferably 5% to 12% by mass, and still more preferably 6% to 11% by mass in the total mass thereof. The content of the non-aromatic hydrophobic crosslinked monomer (B) in the hydrophobic crosslinked copolymer is preferably 40% to 70% by mass and more preferably 55% to 65% by mass in the total mass thereof. The content of the monomer (C) having the reactive functional group in the hydrophobic crosslinked copolymer is preferably 10% to 50% by mass and more preferably 20% to 35% by mass in the total mass thereof. The content of the hydrophobic non-crosslinked monomer (D) in the hydrophobic crosslinked copolymer is preferably 0% to 5% by mass in the total mass thereof. In addition, the ratio (mass ratio) of the monomers (A) to (D) in the hydrophobic crosslinked copolymer is preferably (A):(B):(C):(D) = 5 to 12:55 to 65:20 to 35:0 to 5 (wherein (A) + (B) + (C) + (D) = 100).

A preferable example of procedure for producing the hydrophobic crosslinked copolymer from the monomers (A) to (C) is as follows: a predetermined ratio of the monomers (A), (B) and (C) and benzoyl peroxide as an initiator are added in a 5% by weight of polyvinyl alcohol aqueous solution in a reactor equipped with a stirrer. The resulting mixture is heated with stirring at 80°C for 1 hour under a nitrogen atmosphere to polymerize the monomers (A) to (C). In a case where the monomers (A) to (D) are polymerized, the monomer (D) is further added at a predetermined ratio to the mixture containing the (A) to (C), and the monomers (A) to (D) may be polymerized by heating and stirring in the same manner as described above.

In the packing of the present invention, the type of the cationic functional group present on the surface of the base particle is not particularly limited, and it is preferable to contain a strong cationic group, and more preferable to contain both of a strong cationic group and a weak cationic group.

In the present specification, the strong cationic group means a cationic group that dissociates in a wide range of pH from 1 to 14. That is, the strong cationic group can be kept dissociated (cationized) without being affected by the pH of the aqueous solution.

Examples of the strong cationic group include a quaternary ammonium group. Specific examples include trialkylammonium groups such as a trimethylammonium group, a triethylammonium group, and a dimethylethylammonium group. Examples of the counter ion of the strong cationic group include halide ions such as chloride ion, bromide ion, and iodide ion.

The amount of the strong cationic group present on the surface of the base particle is not particularly limited, but the preferable lower limit per dry weight of the packing is 1 $\mu$eq/g, and the preferable upper limit is 500 $\mu$eq/g. When the amount of the strong cationic group is less than 1 $\mu$eq/g, a DNA retention capacity is weak and the separation performance may deteriorate. When the amount of the strong cationic group exceeds 500 $\mu$eq/g, there are problems in that the retention capacity becomes excessively strong and DNA cannot be eluted easily, and analysis time becomes excessively long.

In the present specification, the weak cationic group means a cationic group having pka of 8 or more. That is, the weak cationic group is affected by the pH of the aqueous solution, and the dissociation state changes. That is, when the pH is higher than 8, the protons of the weak cationic group are dissociated, and the proportion without positive charges increases. On the other hand, when the pH is lower than 8, the weak cationic group becomes protonated, and the proportion having a positive charge increases.

Examples of the weak cationic group include a tertiary amino group, a secondary amino group, and a primary amino group. Among them, a tertiary amino group is desirable.

The amount of the weak cationic group present on the surface of the base particle is not particularly limited, but the preferable lower limit per dry weight of the packing is 0.5 $\mu$eq/g, and the preferable upper limit is 500 $\mu$eq/g. When the amount of the weak cationic group is less than 0.5 $\mu$eq/g, the DNA separation performance may not be improved. When the amount of the weak cationic group exceeds 500 $\mu$eq/g, similar to the strong cationic group, there are problems in that the retention capacity becomes excessively strong and DNA cannot be eluted easily, and analysis time becomes excessively long.

The amount of the strong cationic group or the weak cationic group on the surface of the base particle can be measured by quantifying a nitrogen atom contained in the group. Examples of a method for quantifying the nitrogen atom include a Kjeldahl method. For example, in a case of the base particle containing the strong cationic group and the weak cationic group, first, nitrogen contained in the strong cationic group is quantified after polymerization of the hydrophobic crosslinked copolymer particle and the strong cationic group. Next, a weak cationic group is introduced into the polymer, and the total amount of nitrogen contained in the strong cationic group and the weak cationic group is quantified. From the obtained value, the amount of nitrogen contained in the weak cationic group can be calculated. Thus, based on the quantitative value of the nitrogen atom of the group, the amount of the strong cationic group and the weak cationic group contained in the packing can be adjusted within the above range.

The base particle of the ion-exchange chromatography packing used in the present invention preferably have a polymer layer having the strong cationic group and the weak cationic group on the surface thereof. In the polymer layer having the strong cationic group and the weak cationic group, the strong cationic group and the weak cationic group are preferably derived from independent monomers. Preferably, the base particle of the ion-exchange chromatography packing used in the present invention is obtained by introducing the weak cationic group into a surface of a coated polymer particle consisting of the hydrophobic crosslinked polymer particle and a hydrophilic polymer layer having the strong cationic group copolymerized on the surface of the hydrophobic crosslinked polymer particle.

The hydrophilic polymer having the strong cationic group consists of the hydrophilic monomer having the strong cationic group, and may contain a segment derived from the hydrophilic monomer which has one or more strong cationic groups. That is, as a method for producing the hydrophilic polymer having the strong cationic group, a method for polymerizing a hydrophilic monomer having the strong cationic group alone; a method for copolymerizing two or more hydrophilic monomers having the strong cationic group; and a method for copolymerizing a hydrophilic monomer having the strong cationic group and a hydrophilic monomer not having the strong cationic group are exemplified.

The hydrophilic monomer having the strong cationic group is preferably one having a quaternary ammonium group. Specifically, for example, ethyl methacrylate trimethylammonium chloride, ethyl methacrylate triethylammonium chloride, ethyl methacrylate dimethylethyl ammonium chloride, ethyl methacrylate dimethylbenzyl ammonium chloride, ethyl acrylate dimethylbenzyl ammonium chloride, ethyl acrylate trimethyl ammonium chloride, ethyl acrylate triethyl ammonium chloride, ethyl acrylate dimethylethyl ammonium chloride, acrylamido ethyltrimethyl ammonium chloride, acrylamido ethyltriehyl ammonium chloride and acrylamido ethyldimethylethyl ammonium chloride are exemplified.

As a method for forming a hydrophilic polymer layer having the strong cationic group on the surface of the hydrophobic crosslinked polymer, a method for copolymerizing the hydrophilic monomer having the strong cationic group on the surface of the hydrophobic crosslinked polymer is exemplified.

As a method for introducing a weak cationic group into the surface of the coated polymer particle, a known method can be used. Specifically, examples of a method for introducing a tertiary amino group as the weak cationic group include a method for copolymerizing the hydrophilic monomer having the strong cationic group on the surface of the hydrophobic crosslinked polymer particle having a segment derived from a monomer having a glycidyl group, and then allowing a reagent having a tertiary amino group to react with the glycidyl group; a method for copolymerizing the hydrophilic monomer having the strong cationic group on the surface of the hydrophobic crosslinked polymer particle having a segment derived from the monomer having an isocyanate group, and then allowing a reagent having a tertiary amino group to react with the isocyanate group; a method for copolymerizing the hydrophilic monomer having the strong cationic group and a monomer having a tertiary amino group on the surface of the hydrophobic crosslinked polymer particle; a method for introducing a tertiary amino group to the surface of a hydrophobic crosslinked polymer particle having a hydrophilic polymer layer having a strong cationic group using a silane coupling agent having a tertiary amino group; a method for copolymerizing the hydrophilic monomer having the strong cationic group on the surface of the hydrophobic crosslinked polymer particle having a segment derived from a monomer having a carboxy group, and then condensing a reagent having the carboxy group and a tertiary amino group using carbodiimide; and a method for copolymerizing the hydrophilic monomer having the strong cationic group on the surface of the hydrophobic crosslinked polymer particle having a segment derived from a monomer having an ester bond and hydrolyzing the ester bond, and then condensing a reagent having a carboxy group and a tertiary amino group produced by the hydrolysis using carbodiimide. Among them, preferable examples include a method for copolymerizing a hydrophilic monomer having a strong cationic group on the surface of a hydrophobic crosslinked polymer particle having a segment derived from a monomer having a glycidyl group, and then reacting a reagent having a tertiary amino group with the glycidyl group, a method for copolymerizing a hydrophilic monomer having the strong cationic group on the surface of the hydrophobic crosslinked polymer particle having a segment derived from a monomer having an isocyanate group, and then reacting a tertiary amino group with the isocyanate group.

The reagent having a tertiary amino group reacting with a reactive functional group such as a glycidyl group or an isocyanate group is not particularly limited as long as it is a reagent having a tertiary amino group and a functional group capable of reacting with the reactive functional group. Examples of the functional group capable of reacting with the reactive functional group include a primary amino group and a hydroxyl group. Among them, a group having a primary amino group at a terminal is preferable. Examples of the reagent having a specific tertiary amino group having the functional group include N,N-dimethylaminomethylamine, N,N-dimethylaminoethylamine, N,N-dimethylaminopropylamine, N,N-dimethylaminobutylamine, N,N-diethylaminoethylamine, N,N-diethylaminopropylamine, N,N-diethylaminobutylamine, N,N-diethylaminopentylamine, N,N-diethylaminohexylamine, N,N-dipropylaminobutylamine, and N,N-dibutylaminopropylamine.

In the base particle, the relative positional relationship between the strong cationic group, preferably a quaternary ammonium salt, and the weak cationic group, preferably a tertiary amino group is such that the strong cationic group is preferably located farther from the surface of the hydrophobic crosslinked polymer particle than the weak cationic group, that is, outside. For example, it is preferable that the weak cationic group is within 30 Å from the surface of the hydrophobic crosslinked polymer particle, and the strong cationic group is within 300 Å from the surface of the hydrophobic crosslinked polymer particle and outside the weak cationic group.

The average particle size of the base particles used in the ion-exchange chromatography packing used in the present invention is not particularly limited, and a preferable lower limit is 0.1 $\mu$m and a preferable upper limit is 20 $\mu$m. If the average particle size is less than 0.1 $\mu$m, the pressure of inside of the column may become excessively high, resulting in poor separation. If the average particle size exceeds 20 $\mu$m, a dead volume in the column becomes excessively large, resulting in poor separation. In this specification, the average particle size means a volume average particle size, and can be measured using a particle size distribution measuring device (such as AccuSizer780 manufactured by Particle Sizing Systems).

The ion-exchange chromatography packing of the present invention is suitably used as a packing of the ion-exchange chromatography column for separation and/or detection of methylated DNA. In the separation and/or detection of methylated DNA using the ion-exchange chromatography packing according to the present invention, a target sample DNA may be biologically derived DNA or chemically synthesized DNA. Biological DNA can be extracted, isolated or purified from specimens (for example, tissue cells, blood cells, or cells present in urine, feces, saliva, other body fluids or secretions), cultured cell lines, or the like collected from the living organism. A known technique such as a commercially available DNA extraction kit can be used for extraction, isolation or purification of the sample DNA from a specimen.

The sample DNA is treated with bisulfite and further amplified before being subjected to chromatography using the packing of the present invention. The procedure for treating DNA with bisulfite is well known, and a commercially available kit can also be used. For amplification of DNA, any nucleic acid amplification method such as PCR can be used. There are no particular limitations on the conditions for the amplification reaction, and known methods can be appropriately selected and used according to the sequence, length, amount, or the like of the DNA to be amplified.

In a case where DNA is treated with bisulfite, non-methylated cytosine in the DNA is converted to uracil, but methylated cytosine remains as cytosine. If the bisulfite-treated DNA is amplified by PCR or the like, uracil derived from non-methylated cytosine is further substituted with thymine. As a result, there is a difference in the abundance ratio of cytosine and thymine in the amplification product between methylated DNA and non-methylated DNA. In the ion-exchange chromatography using the packing of the present invention, methylated DNA and non-methylated DNA are separated and/or detected by taking advantage of this difference in base ratio.

In the separation and/or detection of the methylated DNA using the packing of the present invention, the amount of sample injection into the chromatography column is not particularly limited, and may be appropriately adjusted according to the ion-exchange capacity of the column and sample concentration. The lower limit of the amount of sample injected into the chromatography column is preferably 0.1 $\mu$L, more preferably 0.5 $\mu$L, and still more preferably 1 $\mu$L. The upper limit of the amount of sample injected into the chromatography column is preferably 50 $\mu$L, more preferably 25 $\mu$L, and still more preferably 10 $\mu$L. For example, when the inner diameter of the chromatography column is 2.1 mm to 4.6 mm, the amount of sample injected into the column is more preferably 0.1 $\mu$L to 50 $\mu$L, more preferably 0.5 $\mu$L

to 25 $\mu$L, and still more preferably 1 $\mu$L to 10 $\mu$L. The flow rate is preferably from 0.1 mL/min to 3.0 mL/min, and more preferably from 0.5 mL/min to 1.5 mL/min. If the flow rate is slow, improvement of the separation can be expected; however, if the flow rate is excessively slow, it may take a long time for analysis, or the separation performance may be deteriorated due to broad peaks. Conversely, an increase in the flow rate has an advantage in terms of shortening the analysis time, but the peak is compressed, leading to a deteriorate in the separation performance. Therefore, the flow rate is a parameter that is appropriately adjusted depending on the performance of the column, but it is desirable to set the flow rate within the above range. The retention time of each sample in chromatography can be determined in advance by conducting a preliminary experiment on each sample. As a liquid feeding method, a known liquid feeding method such as a linear gradient elution method or a stepwise elution method can be used, but a linear gradient elution method is preferable as the liquid feeding method in the present invention. The magnitude of the gradient (gradient) may be appropriately adjusted in accordance with the separation performance of the column and the properties of the analysis target (DNA) within the range of 0% to 100% of the eluent used for elution.

As the composition of the eluent used for the ion-exchange chromatography using the packing of the present invention, known conditions can be used.

As the buffer used for the eluent, it is preferable to use buffers and organic solvents containing known salt compounds. Specific examples include a Tris-HCl buffer, a TE buffer consisting of Tris and EDTA, and a TBA buffer consisting of Tris, boric acid, and EDTA.

The pH of the eluent is not particularly limited, and the preferable lower limit is 5 and the preferable upper limit is 10. By setting this range, it is considered that the weak cationic group also effectively acts as an ion-exchange group (anion-exchange group). The more preferable lower limit of the pH of the eluent is 6, and the more preferable upper limit of the pH of the eluent is 9.

Examples of the salt contained in the eluent include a salt consisting of halide such as sodium chloride, potassium chloride, sodium bromide, and potassium bromide, and an alkali metal; a salt consisting of a halide such as calcium chloride, calcium bromide, magnesium chloride, magnesium bromide, and an alkaline earth metal; an inorganic acid salt such as sodium perchlorate, potassium perchlorate, sodium sulfate, potassium sulfate, ammonium sulfate, sodium nitrate, and potassium nitrate. Moreover, an organic acid salt such as sodium acetate, potassium acetate, sodium succinate, and potassium succinate can also be used. The salts can be used either alone or in combination.

The salt concentration of the eluent may be appropriately adjusted according to the analysis conditions, and the preferable lower limit is 10 mmol/L, the preferable upper limit is 2000 mmol/L, the more preferable lower limit is 100 mmol/L, and the more preferable upper limit is 1500 mmol/L.

Further, the eluent contains anti-chaotropic ions to further improve the separation performance. The anti-chaotropic ions have properties opposite to those of the kaorotopic ions and have a function of stabilizing a hydration structure. Therefore, there is an effect of strengthening the hydrophobic interaction between the packing and the nucleic acid molecule. The main interaction of the ion-exchange chromatography used in the present invention is electrostatic interaction, and by further utilizing the action of the hydrophobic interaction, the separation performance is enhanced.

Examples of the anti-chaotropic ions contained in the eluent include phosphate ions ($PO_4^{3-}$), sulfate ions ($SO_4^{2-}$), ammonium ions ($NH_4^+$), potassium ions ($K^+$), and sodium ions ($Na^+$). Among these ion combinations, sulfate ions and ammonium ions are preferably used. The anti-chaotropic ions can be used either alone or in combination. Note that a part of the anti-chaotropic ions may contain components of the salt and buffer contained in the eluent. Such a component has both the properties of the salt contained in the eluent or buffer capacity, and the properties of the anti-chaotropic ions, and thus is suitable for the present invention.

The concentration of the anti-chaotropic ions contained in the eluent may be appropriately adjusted according to the analysis target, and is preferably 2000 mmol/L or less as an antichaotropic salt. Specifically, a method for performing gradient elution with the antichaotropic salt concentration in the range of 0 to 2000 mmol/L can be exemplified. Therefore, it is not necessary that the concentration of the antichaotropic salt at the start of the chromatographic analysis is 0 mmol/L, and the concentration of the antichaotropic salt at the end of the analysis is 2000 mmol/L. The method for performing gradient elution may be a low pressure gradient method or a high pressure gradient method, and is preferably a method for eluting while performing precise concentration adjustment by the high pressure gradient method.

The anti-chaotropic ions may be added to only one type of eluent used for elution, or may be added to a plurality of types of eluents. In addition, the anti-chaotropic ions may have both roles of an effect of strengthening the hydrophobic interaction between the packing and DNA or the buffering capacity, and the effect of eluting DNA from the column.

In the ion-exchange chromatography using the packing of the present invention, the column temperature when DNA is analyzed is preferably 30°C or higher, more preferably 40°C or higher, still more preferably 45°C or higher, and even more preferably 60°C or higher. If the column temperature is less than 30°C, the hydrophobic interaction between the packing and DNA becomes weak, and it becomes difficult to obtain a desired separation performance. On the other hand, when the column temperature is higher, methylated DNA and non-methylated DNA are more clearly separated. If the column temperature is 60°C or higher, the difference in the retention time of chromatographic detection signal peaks between the methylated DNA and the non-methylated DNA is widened, and each peak becomes clearer, which makes it possible to detect ethylated DNA more accurately.

On the other hand, if the column temperature of the ion-exchange chromatography is 90°C or higher, double strands of DNA are dissociated, which is not preferable for analysis. Furthermore, if the column temperature is 100°C or higher, the eluent may be boiled, which is not preferable for analysis. Therefore, in the ion-exchange chromatography used in the present invention, the column temperature for analyzing DNA may be 30°C or higher and less than 90°C, preferably 40°C or higher and less than 90°C, more preferably 45°C or higher and less than 90°C, still more preferably 55°C or higher and less than 90°C, further more preferably 60°C or higher and less than 90°C, even more preferably 55°C or higher and 85°C or lower, and even still more preferably 60°C or higher and 85°C or lower.

As described above, after performing the treatment with bisulfite, the amplified DNA has a different sequence depending on the methylation. When the DNA having the different sequence is subjected to the ion-exchange chromatography using the packing of the present invention, a chromatogram showing different signals according to the difference in the sequence is obtained.

More specifically, in the ion-exchange chromatography analysis using the packing of the present invention, the high methylation rate of DNA is reflected in the retention time of the peak of the detection signal. For example, in the ion-exchange chromatography analysis, 100% methylated DNA and non-methylated DNA can be detected as independent peaks (refer to Patent Literature 2). Preferably, the peak of 100% methylated DNA appears with a retention time shorter than that of the non-methylated DNA. Furthermore, the retention time of a peak derived from a partially methylated sample DNA varies depending on the methylation rate. More specifically, the higher the methylation rate of the sample DNA, the more the peak moves toward the 100% methylated DNA peak, that is, the shorter the retention time.

The methylation of the sample DNA can be evaluated by comparing the detection signal of chromatography using the packing of the present invention with the detection signal of the control DNA. As the control DNA, DNA having the same sequence as the sample DNA and a known methylation rate (for example, 0% or 100%) is used. For example, based on the difference in the retention time between the 0% methylation control (negative control) and the 100% methylation control (positive control), the presence or absence of methylation of the sample DNA and the methylation rate can be evaluated. Alternatively, the methylation rate of sample DNA may be calculated based on a calibration curve prepared from data from a plurality of DNAs having different known methylation rates.

As a method for determining the presence or absence of a detection signal peak by chromatography, peak detection using existing data processing software such as LCsolution (Shimadzu), GRAMS/AI (Thermo Fisher Scientific), Igor Pro (WaveMetrics) can be exemplified. In a case where the peak of the detection signal is unclear, the presence or absence of the peak may be detected based on a differential value of the detection signal. The differential value of the detection signal may be automatically calculated by the above-described data processing software, or may be calculated by spreadsheet software (for example, Microsoft (registered trademark) Excel (registered trademark)) or the like.

Examples

Hereinafter, although the present invention will be described in detail with reference to examples; however, this invention is not limited to a following example.

Experiment 1

Base particles of Example 1 and Comparative Examples 1 and 2 were prepared. Table 1 indicates compositions of the base particles. HPLC analysis was performed using the base particle as an ion-exchange chromatography packing.

(1) Production of ion-exchange chromatography packing

(1-1) Preparation of hydrophobic crosslinked polymer particle

For Example 1, the details of the procedure for preparing the hydrophobic crosslinked polymer particle are described below. To 1000 mL of 5% by weight polyvinyl alcohol (produced by The Nippon Synthetic Chemical Industry Co., Ltd.) in a reactor equipped with a stirrer, 20 g of divinylbenzene (produced by Wako Pure Chemical Corporation), 80 g of triethylene glycol dimethacrylate (produced by Shin-Nakamura Chemical Co., Ltd.), 30 g of trimethylol methane triacrylate (produced by Shin-Nakamura Chemical Co., Ltd.), 50 g of glycidyl methacrylate (produced by Wako Pure Chemical Corporation), and 0.8 g of benzoyl peroxide (produced by Kishida Chemical Co., Ltd.) were added. The resulting mixture was heated with stirring at 80°C for 1 hour under a nitrogen atmosphere to generate a polymer. For Comparative Examples 1 and 2, the hydrophobic crosslinked polymer particles were prepared by the same procedure except that the monomer composition was changed as indicated in Table 1.

(1-2) Introduction of strong cationic group

As a hydrophilic monomer having a strong cationic group, 100 g of ethyl methacrylate trimethyl ammonium chloride (produced by Wako Pure Chemical Corporation) was dissolved in ion-exchange water. This was added to the reactor containing the hydrophobic crosslinked polymer particles of (1) and heated at 80°C for 2 hours under stirring in a nitrogen atmosphere so that a monomer having the hydrophobic crosslinked polymer particles and a strong cationic group was polymerized. The obtained product was washed with water and acetone to obtain a coated polymer particle having a hydrophilic polymer layer having a quaternary ammonium group on the surface. The amount of the hydrophilic monomer having a strong cationic group used in the polymerization reaction and the reaction conditions were as indicated in Table 1.

(1-3) Introduction of weak cationic group

10 g of the obtained coated polymer particle was dispersed in 100 mL of ion-exchange water to prepare a pre-reaction slurry. Next, while stirring this slurry, 10 mL of N,N-diethylaminopropylamine (produced by Wako Pure Chemical Corporation), which is a reagent having a weak cationic group, was added and reacted at 70°C for 4 hours. After completion of the reaction, a supernatant was removed using a centrifuge ("Himac CR20G" manufactured by Hitachi, Ltd.) and washed with ion-exchange water. After washing, the supernatant was removed using a centrifuge. This washing with ion-exchange water was further repeated 4 times to introduce the weak cationic groups onto the surface of the coated polymer particles of (2). The amount of the weak cationic groups used in the reaction and the reaction conditions were as indicated in Table 1. Through the above procedure, base particles consisting of the hydrophobic crosslinked polymer particles having quaternary ammonium groups and tertiary amino groups on the surface were obtained.

(1-4) Measurement of particle size

The volume average particle size of the obtained base particle was measured using a particle size distribution measuring device (AccuSizer780 manufactured by Particle Sizing Systems).

[Table 1]

| Material (g) | Base particle | | |
|---|---|---|---|
| | Example 1 | Comparative Example 1 | Comparative Example 2 |
| (A) Divinyl aromatic monomer*1 | 20 | - | - |
| Vinyl aromatic monomer (styrene) | - | - | 20 |
| (B) Non-aromatic hydrophobic crosslinked monomer*2 | 80 | 80 | 80 |
| (B) Non-aromatic hydrophobic crosslinked monomer*3 | 30 | 30 | 30 |
| (C) Monomers having reactive functional groups*4 | 50 | 50 | 50 |
| Strong cationic group*5 | 100 | 100 | 100 |
| (Reaction conditions) | 80°C, 2 hr | 80°C, 2 hr | 80°C, 2 hr |
| Weak cationic group*6 | 10 | 10 | 10 |
| (Reaction conditions) | 70°C, 4 hr | 70°C, 4 hr | 70°C, 4 hr |
| Volume average particle size (μm) | 10 μm | 10 μm | 10 μm |

* 1: Divinylbenzene (Wako Pure Chemical Corporation)
* 2: Triethylene glycol dimethacrylate (produced by Shin-Nakamura Chemical Co., Ltd.)
* 3: Trimethylol methane triacrylate (prepared by Shin-Nakamura Chemical Co., Ltd.)
* 4: Glycidyl methacrylate (produced by Wako Pure Chemical Corporation)
* 5: Ethyl methacrylate trimethylammonium chloride (produced by Wako Pure Chemical Corporation)
* 6: N,N-diethylaminopropylamine (produced by Wako Pure Chemical Corporation)

(2) Detection of methylated DNA by HPLC

(2-1) Preparation of sample DNA

Synthetic DNA constructed based on the DNA sequence of the FAM150A gene (384 bp, having 39 CpG sites) was used as sample DNA.

Sample 1: Methylated DNA: All 39 CpG site bases are CG
Sample 2: Non-methylated DNA: All 39 CpG site bases are TG

The samples 1 and 2 correspond to bisulfite-treated products of methylated DNA (methylation rate 100%) and non-methylated DNA (methylation rate 0%) of the FAM150A gene, respectively. A reaction solution obtained by PCR amplification of the DNAs of the samples 1 and 2 (15 ng/100 μL) was used as a sample for HPLC analysis.

(2-2) HPLC analysis conditions

HPLC analysis was performed using the base particle obtained in (1) as an ion-exchange chromatography packing. The base particles of Example 1 and Comparative Examples 1 and 2 were each packed into a stainless steel column (inner diameter of 4.6 mm × length of 20 mm) of a liquid chromatography system. By using the obtained anion-exchange column, the ion-exchange chromatography was performed under the following conditions.
System: LC-20A series (produced by Shimadzu Corporation)
Eluent:

Eluent A 25 mmol/L Tris-HCl buffer (pH 7.5)
Eluent B 25 mmol/L Tris-HCl buffer (pH 7.5)
+ 2 mol/L ammonium sulfate

Analysis time: 15 minutes
Elution method: The mixing ratio of eluent B was increased linearly under the following gradient conditions.
0 min (eluent B 40%) → 10 min (eluent B 100%)
Flow rate: 1.0 mL/min
Sample: PCR amplification products of samples 1 and 2 described in (1)
Sample injection volume: 5 μL
Column temperature: 70°C
Detection wavelength: 260 nm

(2-3) Analysis results

A chromatogram of the sample DNA obtained by HPLC using the base particles of Example 1 and Comparative Examples 1 and 2 is illustrated in Fig. 1. In the HPLC chromatogram using the packing of Example 1, the peaks of methylated DNA (sample 1) and non-methylated DNA (sample 2) are clearer than those of Comparative Examples 1 and 2, and the difference in both retention times was increased. According to the following formula, the degree of separation of the methylated DNA and the non-methylated DNA peak was calculated. As indicated in Table 2, in a case where the base particles of Example 1 were used, the degree of separation was significantly improved. From this, it was shown that the base particle of Example 1 has high separation performance for methylated DNA and non-methylated DNA.

```
Degree of separation = 1.18 × (Retention time of non-methylated DNA - Retention time of methylated DNA)/(Half

width of non-methylated DNA + Half width of methylated DNA)
```

[Table 2]

|  | Example 1 | Comparative Example 1 | Comparative Example 2 |
|---|---|---|---|
| Degree of separation | 1.84 | 1.02 | 0.99 |

Experiment 2

Base particles 1 to 3 having different monomer (A) contents as indicated in Table 3 were produced in the same procedure as in Experiment 1 (1) (volume average particle size 10 μm). By using the obtained base particle as an ion-exchange chromatography packing, HPLC analysis was performed in the same procedure as in Experiment 1 (2), and the degree of separation between the peaks of methylated DNA and non-methylated DNA was calculated. The results are indicated in Table 3.

[Table 3]

| Material (g) | Base particle 1 | Base particle 2 | Example 1 | Base particle 3 |
|---|---|---|---|---|
| (A) Divinyl aromatic monomer*1 | 5 | 10 | 20 | 30 |
| (B) Non-aromatic hydrophobic crosslinked monomer*2 | 80 | | | |
| (B) Non-aromatic hydrophobic crosslinked monomer*3 | 30 | | | |
| (C) Monomers having reactive functional groups*4 | 50 | | | |
| Ratio (%) of (A) in hydrophobic crosslinked polymer particle | 3.0 | 5.9 | 11.1 | 15.8 |
| Strong cationic group*5 | 100 | | | |
| Weak cationic group*6 | 10 | | | |
| Degree of separation | 1.15 | 1.49 | 1.84 | 1.28 |

* 1: Divinylbenzene (Wako Pure Chemical Corporation)
* 2: Triethylene glycol dimethacrylate (produced by Shin-Nakamura Chemical Co., Ltd.)
* 3: Trimethylol methane triacrylate (produced by Shin-Nakamura Chemical Co., Ltd.)
* 4: Glycidyl methacrylate (produced by Wako Pure Chemical Corporation)
* 5: Ethyl methacrylate trimethylammonium chloride (produced by Wako Pure Chemical Corporation)
* 6: N,N-diethylaminopropylamine (produced by Wako Pure Chemical Corporation)

As indicated in Table 3, in the HPLC using the base particles 1 to 3, the degree of separation of methylated DNA and non-methylated DNA was improved as compared with Comparative Examples 1 and 2. From this, it was shown that the base particles 1 to 3 have high separation performance for methylated DNA and non-methylated DNA.

**Claims**

1. An ion-exchange chromatography packing for separation and/or detection of methylated DNA, comprising a base particle consisting of a hydrophobic crosslinked copolymer particle and having a cationic functional group on a surface, wherein a hydrophobic crosslinked copolymer contains a divinyl aromatic monomer.

2. The ion-exchange chromatography packing according to claim 1, wherein the divinyl aromatic monomer is selected from the group consisting of divinylbenzene, divinylnaphthalene, divinylanthracene, divinyltoluene, divinylxylene, and divinylbiphenyl.

3. The ion-exchange chromatography packing according to claim 1 or 2, wherein the hydrophobic crosslinked copolymer contains 3% to 15% by mass of the divinyl aromatic monomer in a total mass.

4. The ion-exchange chromatography packing according to any one of claims 1 to 3, wherein the hydrophobic crosslinked copolymer contains the divinyl aromatic monomer, a non-aromatic hydrophobic crosslinked monomer having two or more vinyl groups, and a monomer having a reactive functional group.

5. The ion-exchange chromatography packing according to claim 4, wherein the reactive functional group is a glycidyl group or an isocyanate group.

6. The ion-exchange chromatography packing according to claim 4 or 5, wherein the non-aromatic hydrophobic crosslinked monomer having two or more vinyl groups is at least one selected from the group consisting of diethylene glycol dimethacrylate, triethylene glycol dimethacrylate, tetraethylene glycol dimethacrylate, trimethylol methane trimethacrylate, tetramethylol methane trimethacrylate, diethylene glycol diacrylate, triethylene glycol diacrylate, tetraethylene glycol diacrylate, trimethylol methane triacrylate, and tetramethylol methane triacrylate.

7. The ion-exchange chromatography packing according to any one of claims 1 to 6, wherein the hydrophobic crosslinked copolymer contains 3% to 15% by mass of the divinyl aromatic monomer, 40% to 70% by mass of the non-aromatic hydrophobic crosslinked monomer, and 10% to 50% by mass of the monomer having the reactive functional group in the total mass.

8. The ion-exchange chromatography packing according to any one of claims 1 to 7, wherein the hydrophobic crosslinked copolymer further contains 0% to 5% by mass of a hydrophobic non-crosslinked monomer in the total mass.

9. The ion-exchange chromatography packing according to any one of claims 1 to 8, wherein the cationic functional groups are a strong cationic group and a weak cationic group.

10. The ion-exchange chromatography packing according to claim 9, wherein the strong cationic group is a quaternary ammonium group and the weak cationic group is a tertiary amino group.

11. The ion-exchange chromatography packing according to claim 9 or 10, wherein the base particle has a polymer layer having the strong cationic group and the weak cationic group on the surface.

12. An ion-exchange chromatography column for separation and/or detection of methylated DNA containing the ion-exchange chromatography packing according to any one of claims 1 to 11.

13. A method for separating and/or detecting methylated DNA using the ion-exchange chromatography packing according to any one of claims 1 to 11.

[Figure 1]

## INTERNATIONAL SEARCH REPORT

| International application No. |
|---|
| PCT/JP2018/031539 |

**A. CLASSIFICATION OF SUBJECT MATTER**

Int.Cl. B01J20/285(2006.01)i, B01D15/36(2006.01)i, B01J41/04(2017.01)i, B01J41/14(2006.01)i, B01J41/20(2006.01)i, B01J47/00(2017.01)i, C12N15/00(2006.01)i, C12Q1/68(2018.01)i, G01N30/02(2006.01)i, G01N30/88(2006.01)i

According to International Patent Classification (IPC) or to both national classification and IPC

**B. FIELDS SEARCHED**

Minimum documentation searched (classification system followed by classification symbols)

Int.Cl. B01J20/285, B01D15/36, B01J41/04, B01J41/14, B01J41/20, B01J47/00, C12N15/00, C12Q1/68, G01N30/02, G01N30/88

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | IGLESIAS, T. et al., "Anion exchange chromatography for the determination of 5-methyl-2'deoxycytidine: application to cisplatin-sensitive and cisplatin-resistant ovarian cancer cell lines", ANALYTICAL AND BIOANALYTICAL CHEMISTRY, 2015, vol. 407, no. 9, pp. 2423-2431 | 1-2, 12-13 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 November 2018 (15.11.2018) | 27 November 2018 (27.11.2018) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/031539

C (Continuation).    DOCUMENTS CONSIDERED TO BE RELEVANT

| Category* | Citation of document, with indication, where appropriate, of the relevant passages | Relevant to claim No. |
|---|---|---|
| Y | WO 2014/136930 A1 (SEKISUI MEDICAL CO., LTD.) 12 September 2014, claims, paragraphs [0021]-[0040], [0074]-[0076] & US 2016/0138097 A1, claims, paragraphs [0047]-[0066], [0100]-[0102] & EP 2966179 A1 & CN 105074010 A | 1-13 |
| Y | WO 2016/117572 A1 (HITACHI CHEMICAL CO., LTD.) 28 July 2016, claims 1, 5, 12, paragraphs [0019], [0083]-[0087] & US 2017/0341058 A1, claims, paragraphs [0040], [0114]-[0124] & EP 3248678 A1 | 1-13 |
| A | WO 2016/205233 A2 (CEPHEID) 22 December 2016, claims & JP 2018-524978 A & US 2017/0137871 A1 | 1-13 |
| A | WIEDER, W. et al., "Monolithic poly (glycidy 1 methacrylate-co-divinylbenzene) capillary columns functionalized to strong anion exchangers for nucleotide and oligonucleotide separation", JOURNAL OF SEPARATION SCIENCE, 2006, vol. 29, pp. 2478-2484 | 1-13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012108516 A

- WO 2014136930 A